# EUROPEAN PATENT APPLICATION

(11) **EP 4 385 442 A1**
(43) Date of publication of application: **19.06.2024**
(21) Application number: 23217072.0
(22) Date of filing: 15.12.2023
(51) Int. Cl.: A61B 18/14, A61B 5/0538

(54) **CATHETER WITH PULL RING COUPLER**

(30) Priority: 16.12.2022 US 202218067115
(71) Applicant: Biosense Webster (Israel) Ltd., 2066717 Yokneam (IL)
(72) Inventor: VAN NIEKERK, Pieter Emmelius, Irvine, 92618 (US); SOLIS, Mario A., Irvine, 92618 (US); LAZO, Erik, Irvine, 92618 (US); HENRIQUEZ, Jamie Lynn, Irvine, 92618 (US); FUENTES-ORTEGA, Cesar, Irvine, 92618 (US)
(74) Representative: Carpmaels & Ransford LLP

(57) **Abstract**

A multi-electrode catheter is presented herein having a puller wire with a distal end bonded to a coupler tube between a catheter shaft body and end effector. Because the puller wire is bonded to the coupler tube, a distal portion of the catheter body shaft, at least up to a proximal end of the coupler tube, can be deflected when the puller wire is retracted. The coupler tube may also be flexible and be deflectable when the puller wire is retracted. The coupler tube may bend with a radius of curvature that is greater than, equal to, or less than a radius of curvature of the distal portion of the catheter body shaft, depending on design considerations of the catheter. The coupler tube may be made from a flexible material and/or may include sidewall slits which facilitate bending of the coupler tube.

## Description

### FIELD OF INVENTION

The present disclosure relates to a deflectable catheter for use in the vessel of a patient for the purpose of diagnosing or treating the patient, such as mapping tissue and/or ablating tissue using radio frequency (RF), irreversible electroporation (IRE), or other sources of energy.

### BACKGROUND

Cardiac arrythmias are characterized as phenomenon that occurs when regions of cardiac tissue abnormally conduct electric signals to adjacent tissue. Examples of cardiac arrythmias can include, but not be limited to atrial fibrillation, supraventricular tachyarrhythmias, and the like. Resultantly, this phenomenon can disrupt the normal cardiac cycle, thus causing asynchronous cardiac cycles or rhythms. The source of the undesired conduction of adjacent cardiac tissue can be attributed to tissue disposed in either an atria or a ventricle. Consequently, if the cardiac arrythmia remains unresolved, the unwanted signals can proliferate through the cardiac tissue, which can result in continued initiation or persistence of arrythmias.

Medical procedures to treat cardiac arrythmias generally include two steps: (1) mapping the electrical properties of the endocardium; and (2) selectively ablating cardiac tissue based on the mapped endocardium. To create the endocardium schematic, electrical activity can be measured at specific locations in the heart, which can be performed by advancing a catheter containing one or more electrical sensors into the heart to acquire the electrical activity data. Once the electrical data is obtained, the practitioner can create a schematic that can then be utilized to designate target areas where the ablation will be performed.

Given the tortuous nature of cardiac vasculature and the variety of cardiac tissue textures, it is desirable for the catheter to be adaptable to different tissue surfaces and deflectable to reduce the potential for causing trauma to a patient's vasculature and cardiac tissues. Additionally, for greater mapping resolution, it is also desirable for the catheter to support the use of several electrodes that can sense electrical activity in cardiac tissue, specifically an area that can be on the order of a square centimeter. Thus there is a need for systems, devices and methods that can provide improvements to catheter designs, specifically providing improvements in flexibility while maintaining an atraumatic profile when utilized within highly tortuous and textured anatomical environments.

### SUMMARY

A catheter can include an elongated shaft that extends along a longitudinal axis, an electrode assembly that is disposed distal of the elongated shaft, a coupler tube that is disposed between the elongated shaft and the electrode assembly, and a puller wire that extends through the elongated shaft. The puller wire can include a distal end that can be joined to a sidewall of the coupler tube so that translation of the puller wire along the longitudinal axis deflects a distal portion of the elongated shaft away from the longitudinal axis so that the distal portion defines a first radius of curvature and the coupler tube defines a second radius of curvature.

The distal portion of the elongated shaft, that is deflectable from the longitudinal axis by the puller wire, can include a length of the elongated shaft up to a proximal end of the coupler tube.

The puller wire can also be configured to deflect at least a portion of the coupler tube. The first radius of curvature can be less than the second radius of curvature; the first radius of curvature can be approximately equal to the second radius of curvature; or the first radius of curvature can be greater than the second radius of curvature.

The coupler tube can include a plurality of sidewall slits that can be configured to facilitate deflection of the coupler tube. The sidewall slits can include a plurality of slits each orthogonal to the longitudinal axis. The sidewall slits can include a plurality of slits aligned along the longitudinal axis with the distal end of the puller wire.

The coupler tube can include a material with a durometer that can be configured to allow the coupler tube to deflect.

The distal end of the puller wire can be affixed to an inner sidewall of the coupler tube.

The coupler tube can include a single lumen.

The elongated shaft can include a distal extension that can be disposed within a proximal portion of the coupler tube.

The electrode assembly can include a support frame which can include a proximal portion that can be disposed within a distal portion of the coupler tube.

The catheter can further include a disk that can be affixed within the distal portion of the coupler tube. The disk can be joined to the proximal portion of the support frame and can include one or more lumens with electrical conductors extending therethrough.

The catheter can further include elongated conductors that can extend from the electrode assembly, the coupler tuber, and the elongated shaft.

The catheter can further include an electromagnetic position sensor that can be disposed within the coupler tube.

The catheter can further include a pair of puller wires with each extending through the elongated shaft. Each of the puller wires can include a distal end affixed to the coupler tube. Each of the puller wires can be configured to deflect a distal portion of the elongated shaft from the longitudinal axis. The distal ends of the puller wires can be affixed on opposite sides of the coupler tube.

The electrode assembly can include an array of electrodes that can be arranged in a planar grid, in a sphere, and/or in a helical or circular shape. The electrode assembly can include a plurality of radial spines. Alternatively, the electrode assembly can include a single irrigated tip electrode and a contact force sensor.

The catheter can include a handle that disposed proximal of the elongated shaft configured to provide tension to the puller wire.

A proximal portion of the elongated shaft can include a stiffening tube configured to inhibit deflection of the proximal portion of the elongated shaft by the puller wire.

Another example catheter can include an elongated shaft that extends along a longitudinal axis, an electrode assembly disposed distal of the elongated shaft, a coupler tube disposed between the elongated shaft and the electrode assembly, and a puller wire extending through the elongated shaft. The puller wire can include a distal end affixed to the coupler tube and configured to deflect at least a portion of the coupler tube and a distal portion of the elongated tube.

The puller wire can be configured to deflect the distal portion of the elongated shaft with a radius of deflection less than a radius of deflection of the coupler tube, approximately equal to a radius of deflection of the coupler tube, or greater than a radius of deflection of the coupler tube.

The coupler tube can include a plurality of sidewall slits configured to facilitate deflection of the coupler tube.

The sidewall slits can include a plurality of slits each orthogonal to the longitudinal axis. The sidewall slits can include a plurality of slits aligned along the longitudinal axis with the distal end with the distal end of the puller wire.

The coupler tube can include a material with a durometer configured to allow the coupler tube to deflect.

The distal end of the puller wire can be affixed to an inner sidewall of the coupler tube.

An example method of manufacturing a catheter can include the following steps executed in a variety of orders and with interleaving steps as understood by a person skilled in the art. The method can include: joining a puller wire to a sidewall of a coupler tube; affixing an electrode assembly to a distal end of the coupler tube; and affixing an elongated shaft to a proximal end of the coupler tube so that the distal portion defines a first radius of curvature and the coupler tube defines a second radius of curvature.

The puller wire can be configured to deflect a distal portion of the elongated shaft including a length of the elongated shaft up to a proximal end of the coupler tube. The puller wire can be configured to deflect at least a portion of the coupler tube.

The method can further include selecting a material durometer of the coupler tube to allow the coupler tube to deflect. The method can further include cutting sidewall slits in the coupler tube that can be configured to facilitate deflection of the coupler tube. The method can further include joining the puller wire to the sidewall of the coupler tube, which can include welding the puller wire through the sidewall of the coupler tube.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of a distal portion of a first example catheter according to aspects of the present disclosure.
FIG. 2 is an illustration of a distal portion of a second example catheter according to aspects of the present disclosure.
FIG. 3 is an illustration of a distal portion of a third example catheter according to aspects of the present disclosure.
FIG. 4 is an illustration of a fourth example catheter according to aspects of the present disclosure
FIG. 5 is an illustration of aspects of an example coupler tube at a distal portion of a shaft of the catheter according to aspects of the present invention.
FIG. 6 is an illustration of further aspects of an example coupler tube at a distal portion of a shaft of a catheter according to aspects of the present invention.
FIG. 7 is an illustration of a catheter having an end effector at a distal portion of the catheter and a proximal handle at a proximal portion of the catheter according to aspects of the present invention.
FIG. 8 is illustration of a system for performing mapping and ablative procedures, which is constructed and operative according to aspects of the invention.

### DETAILED DESCRIPTION

The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

As used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

As used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the features presented herein may be combined will be readily apparent to those skilled in the pertinent art in view of the disclosure. Such modifications and variations are intended to be included within the scope of the claims.

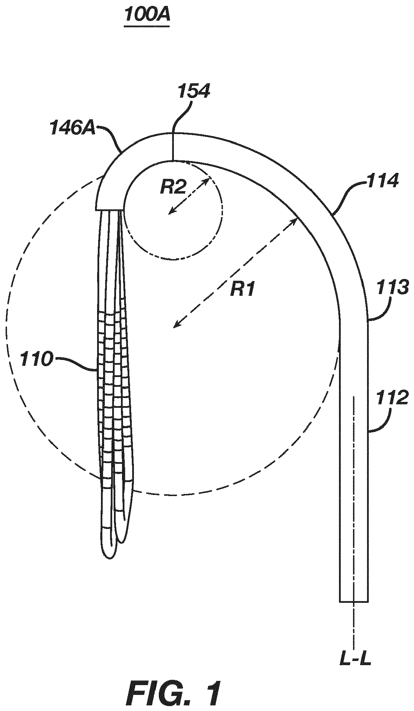

FIGs. 1-4 are illustrations of four example catheters: a first example catheter 100A, a second example catheter 100B, a third example catheter 100C, and a fourth example catheter 100D. The example catheters 100A, 100B, 100C, 100D can have several design variations including compatible features of other embodiments and examples presented herein, compatible features of publications incorporated herein by reference, and other compatible features as understood by a person skilled in the pertinent art. Each example catheter 100A, 100B, 100C, 100D includes a distal portion 114 having a first radius of curvature R1. For the sake of illustration, the first radius of curvature R1 is equal across the four example catheters 100A, 100B, 100C, 100D and the distal portion 114 of the shaft bends about 90° through an arc defined by the first radius of curvature R1; however, the first radius of curvature R1 can be more or less than illustrated and the bend of the distal portion 114 of the shaft can be more or less than illustrated to achieve desired geometry of the shaft. The shaft of each of the catheters 100A, 100B, 100C, 100D includes a proximal section 112 and a transition 113 between the proximal section 112 and the distal portion 114. The proximal section 112 is aligned with a longitudinal axis L-L.

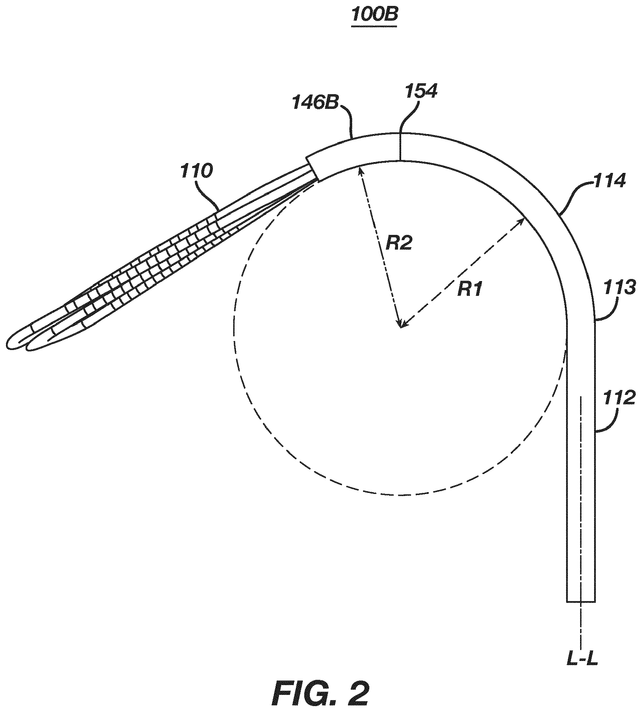

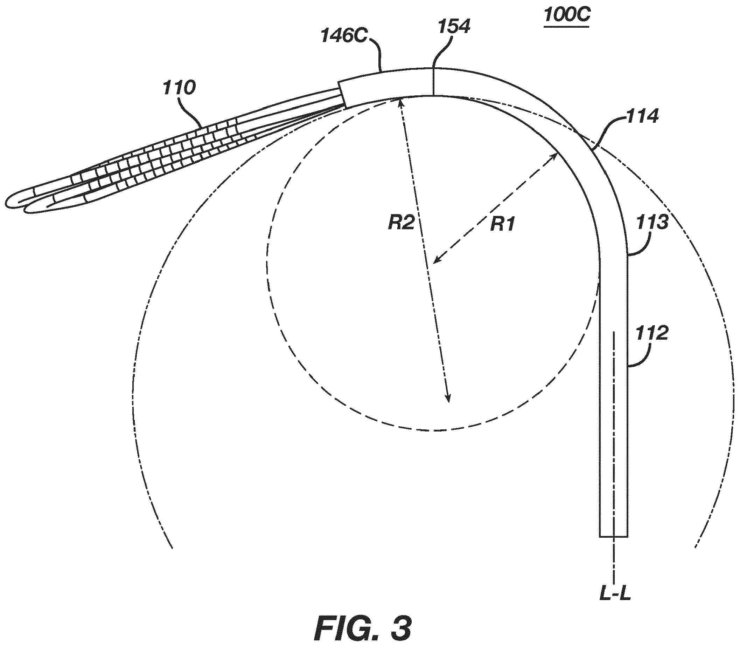

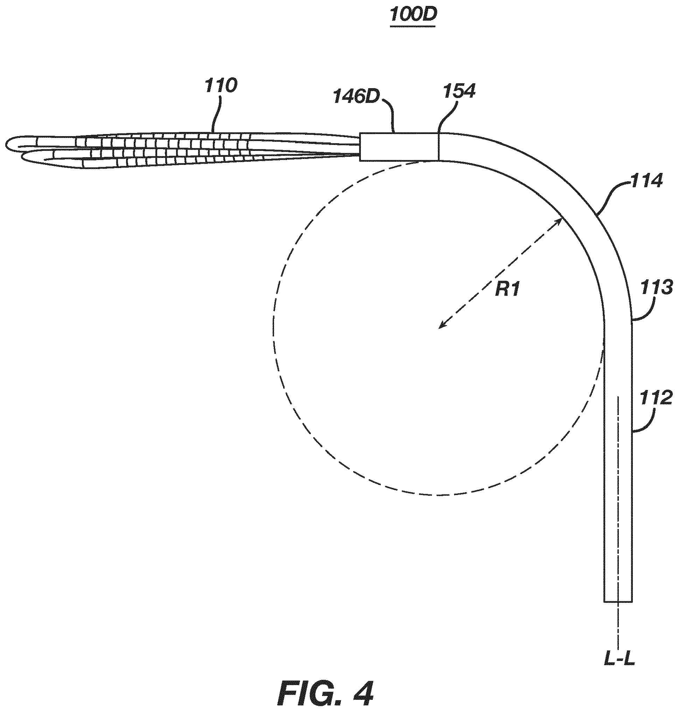

Each example catheter 100A, 100B, 100C, 100D includes a respective coupler tube 146A, 146B, 146C, 146D. The first, second, and third example catheters 100A, 100B, 100C have a respective second radius of curvature R2 of the respective coupler tube 146A, 146B, 146C. The first example catheter 100A (FIG. 1) has a second radius of curvature R2 less than the first radius of curvature R1. The second example catheter 100B (FIG. 2) has a second radius of curvature R2 equal to the first radius of curvature R1. The third example catheter 100C (FIG. 3) has a second radius of curvature R2 greater than the first radius of curvature R1. The fourth example catheter 100D (FIG. 4) includes a substantially straight coupler tube 146D. The curvatures presented are purely for the sake of illustration, and example catheters having any degree of curvatures within the ranges of curvatures presented herein, and as otherwise understood to a person skilled in the pertinent art upon reading the present disclosure are within the scope of the present disclosure.

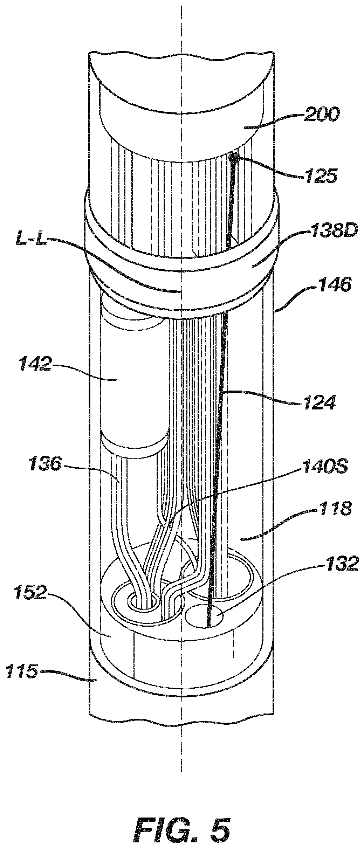

FIGs. 5-6 are illustrations of aspects of a coupler tube 146 with features that can be utilized in the examples illustrated FIGs. 1-4 and other example catheters presented herein.

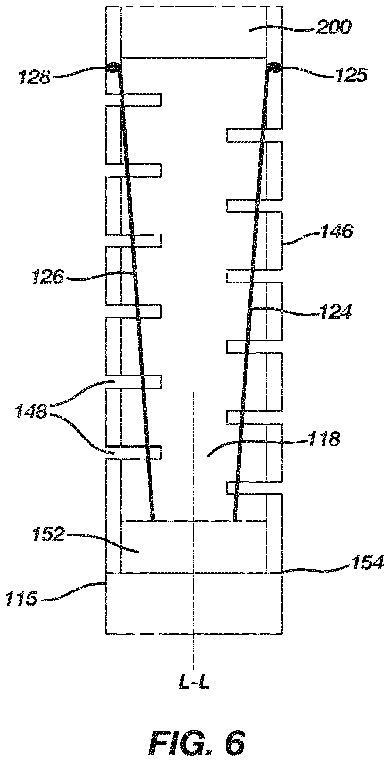

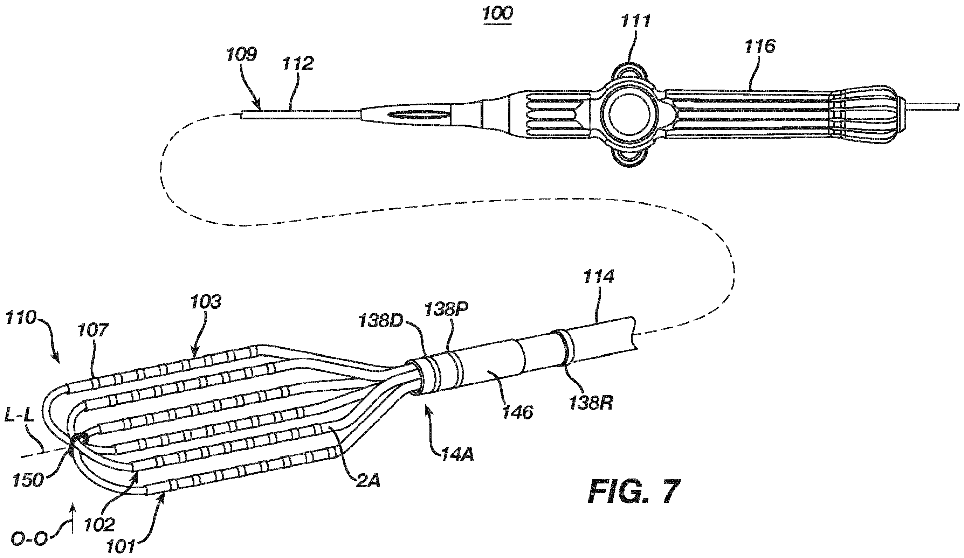

FIG. 7 is an illustration of an example catheter 100 that can include features of the example catheters 100A, 100B, 100C, 100D illustrated in FIGs. 1-4 and/or features of the coupler tube 146 illustrated in FIGs. 5-6. The catheter 100 can have an elongated shaft 109. The catheter 100 can also have a handle 116. The handle 116 can be disposed proximal to the elongated shaft 109 and can be configured to provide tension to a first puller wire 124 or second puller wire 126 (FIG. 5 & FIG. 6). The proximal portion 112 of the elongated shaft 109 can extend along a longitudinal axis L-L. The elongated shaft 109 forms a tubular catheter body sized and otherwise configured to traverse vasculature. The proximal portion 112 of the elongated shaft 109 can also include a stiffening tube. The stiffening tube can be configured to inhibit deflection of the proximal portion 112 of the elongated shaft 109. The proximal portion 112 of the elongated shaft 109 can further be configured similarly as disclosed in U.S. Patent Publication No. 2021/0369339, incorporated by reference herein. The shaft 109 can additionally, or alternatively include features understood by a person skilled in the pertinent art.

The distal portion 114 of the elongated shaft 109 can transition to the coupler tube 146, which can extend distally from a distal end 115 of the elongated shaft 109 and be disposed in a proximal direction in relation to the end effector 110.

The distal portion 114 of the elongated shaft 109 can be configured to be deflectable and can transition to the coupler tube 146. This transition can be disposed at the intersection of the distal end 115 of the elongated shaft 109 and the proximal end 154 of the coupler tube 146 (FIGs. 1-4). Additionally, the catheter 100 can include a first puller wire 124 that can extend through the elongated shaft 109 and can be joined to a sidewall of the coupler tube 146 (FIGs. 5-6). The joining of the first puller wire 124 to the sidewall of the coupler tube 146 can allow for displacement of the first puller wire 124 along the longitudinal axis L-L to deflect the distal portion of the elongated shaft 109 away from the longitudinal axis L-L (FIGs. 5-6). The deflection of the distal portion 114 of the elongated shaft 109 away from the longitudinal axis L-L can define the first radius of curvature R1 and the coupler tube 146 can define the second radius of curvature R2.

The first example catheter 100A, illustrated in FIG. 1, can be distinguished by the first radius of curvature R1 being greater than the second radius of curvature R2. The first radius of curvature R1 can be measured radially based on a degree of curvature of the distal portion 114 of the elongated shaft 109 away from the longitudinal axis L-L. The curve of the distal portion 114 approximates an arc section of an imaginary first circle C1 having the first radius R1. The distal portion 114 of the elongated shaft 109 is deflectable from the longitudinal axis L-L including a length of the distal portion 114 up to the proximal end of the coupler tube 146A, i.e. up to the transition between the proximal end 154 of the coupler tube 146A and the distal end 115 of the distal portion 114. The curve of the coupler tube 146A approximates an arc section of an imaginary second circle C2 having a second radius R2. The second radius of curvature R2 can allow for the elongated shaft 109 to make a total angle of curvature that is greater than a comparable catheter having an inflexible coupler tube. As illustrated, the shaft and coupler tube collectively have a curvature of approximately 180° so that the end effector is aligned substantially parallel to the longitudinal axis L-L.

The second example catheter 100B, illustrated in FIG. 2 can be distinguished by the first radius of curvature R1 being approximately equal to the second radius of curvature R2. Similar to as illustrated in FIG. 1, the curve of the distal portion 114 of the elongated shaft 109 of the second example catheter 100B in FIG. 2 approximates an arc section of an imaginary first circle C1 having the first radius R1. The distal portion 114 of the elongated shaft 109 is deflectable from the longitudinal axis L-L including a length of the distal portion 114 up to the proximal end of the coupler tube 146B, i.e. up to the transition between the coupler tube 146B and the distal portion 114. As illustrated, the arc formed by the distal portion 114 and the arc formed from the coupler tube 146B approximate the same imaginary circle C1. As illustrated, the shaft and coupler tube collectively have a curvature between 90° and 180°.

The third example catheter 100C, illustrated in FIG. 3, can be distinguished by the first radius of curvature R1 being less than the second radius of curvature R2. Similar to as illustrated in FIG. 1, the curve of the distal portion 114 of the elongated shaft 109 of the third example catheter 100C in FIG. 3 approximates an arc section of an imaginary first circle C1 having the first radius R1. The distal portion 114 of the elongated shaft 109 is deflectable from the longitudinal axis L-L including a length of the distal portion 114 up to the proximal end 154 of the coupler tube 146D, i.e. up to the transition between the coupler tube 146C and the distal portion 114 of the shaft 109. The curve of the coupler tube 146C approximates an arc section of an imaginary second circle C2 having a second radius R2 that is greater than the first radius R1. As illustrated, the shaft and coupler tube collectively have a curvature between 90° and 180°, and the curvature is less than the curvature of the shaft and coupler tube illustrated in FIG. 2.

The fourth example catheter 100D, illustrated in FIG. 4, can be distinguished by having a substantially linear coupler tube 146D. Similar to as illustrated in FIG. 1, the curve of the distal portion 114 of the elongated shaft 109 of the fourth example catheter 100D in FIG. 4 approximates an arc section of an imaginary first circle C1 having the first radius R1. The distal portion 114 of the elongated shaft 109 is deflectable from the longitudinal axis L-L including a length of the distal portion 114 up to the proximal end 154 of the coupler tube 146D, i.e. up to the transition between the coupler tube 146D and the distal portion 114 of the shaft 109. In contrast to FIGs. 1-3, the connector tube 146D in FIG. 4 has no degree of curvature. The coupler tube 146D is approximately linear, which can result in the elongated shaft 109 being configured to make a total angle of curvature that is less than a comparable catheter having a coupler tube that is configured to bend by pulling a puller wire. The coupler tube 146D can be configured to be inflexible, or rigid. As illustrated, the elongated shaft 109 can have a curvature of approximately 90° so that the end effector is aligned substantially orthogonal to the longitudinal axis L-L. Note that while the coupler tube 146D is inflexible, the distal portion 114 of the elongated shaft 109 is flexible up to the transition 154 between the distal portion 114 and the coupler tube 146D, which can result in a total angle of curvature that is greater than a comparable catheter having a short distance (e.g. a few millimeters) of the distal section being inflexible near the transition 154 between the distal section and the coupler tube.

FIG. 5 is an illustration of a section of the catheter 100 including the coupler tube 146 in greater detail. The distal portion 114 of the elongated shaft 109 is coupled to the end effector 110 (FIG. 7) with the coupler tube 146.

The coupler tube 146 can include a tubular insert 200 for connection of loop members 101, 102, 103 to provide electrical connection through the distal portion 114 of the elongated shaft 109 (FIG. 7). The insert 200 can be in the shape of a disk and can include openings therethrough to receive portions of the end effector. The insert 200 can be configured similarly to the tubular insert 200 illustrated in FIG. 4B of U.S. Patent Publication No. 2021/0369339, incorporated by reference herein.

The coupler tube 146 preferably includes a single central lumen 118 to allow for greater flexibility of the coupler tube 146. Alternatively, the coupler tube 146 can include multiple lumens. The coupler tube 146 can be shaped to house various components such as an electromagnetic position sensor 142, ring electrodes 138D, lead wires 140S to electrodes of the end effector, a cable 136 for the electromagnetic position sensor 142, etc.

A distal portion of a puller wire 124 can extend through the coupler tube 146. A distal end of the puller wire can be coupled to a sidewall of the coupler tube 146 with an anchor 125. The anchor 125 can include a weld, glue, or other suitable structure as understood by a person skilled in the pertinent art. A distal portion of a second puller wire 126 (FIG. 6) can extend through the coupler tube 146 and a distal end of the second puller wire 126 can be coupled to a sidewall of the coupler tube by a second anchor 128. Only the first anchor 125 for the first puller wire 124 is visible in FIG. 5 for the sake of illustration. The catheter 100 can include only one puller wire 124 for unidirectional bending. The catheter 100 can include two puller wires for bidirectional bending. As an alternative to being coupled to the sidewall, the distal end of the first puller wire 124 and/or the distal end of the second puller wire 126 can be coupled to the insert 200. In this example, the puller wire(s) can be affixed to the insert 200 (in a previous assembly), after which the insert 200 can be affixed to the coupler tube 146.

The elongated shaft 109 can include a distal extension 152 that extends into the coupler tube 146 through the proximal end 154 of the coupler tube 146. The coupler tube 146 can be affixed to the distal extension 152 of the distal portion 114 of the elongated shaft 109 of the catheter 100 by glue or the like. The distal extension 152 can include a lumen 132 to receive the puller wire 124. The distal extension 152 can include additional lumens configured to receive the cable 136, lead wires 140S, and the second puller wire 126 (FIG. 6).

FIG. 6 is an illustration of a cross-sectional view of the coupler tube 146 with various components housed within the coupler tube 146 omitted for the sake of illustration. The coupler tube 146 can include a low durometer/high flexibility material, which can be configured to allow the coupler tube 146 to deflect. Additionally, or alternatively, the coupler tube 146 can include a plurality of sidewall slits 148 that can be oriented orthogonally to the longitudinal axis L-L, as illustrated. The second puller wire 126 is illustrated opposite the first puller wire 125. Like the first puller wire 124, the second puller wire 126 can be coupled to the sidewall of the coupler tube 146 with the second anchor 128, which can be disposed opposite the first anchor 125. The first puller wire 124 and the second puller wire 126 can both be configured to deflect a length of the elongated shaft 109 up to a proximal end 154 of the coupler tube 146 and at least some section of the coupler tube 146 from the longitudinal axis L-L. Both the first puller wire 124 and the second puller wire can extend through the distal extension 152 of the distal portion 114 of the elongated shaft 109 and therein through the proximal end 154 of the coupler tube 146.

When the second puller wire 126 is retracted, the distal portion 114 of the elongated shaft 109 can bend to the left, forming the first radius of curvature R1 as illustrated in FIGs. 1-3. The sidewall slits 148 can cause the coupler tube 146 to be flexible so that the coupler tube 146 bends to the left simultaneously with the distal portion 114 as the second puller wire 126 is retracted. The dimensions of the sidewall slits 148 affect the flexibility of the coupler tube 146 as understood by a person skilled in the pertinent art. Therefore, the dimensions of the sidewall slits 148 can be selected to result in the coupler tube have a predetermined second radius of curvature R2 for a given first radius of curvature R1 of the distal portion 114 of the elongated shaft 109. For instance, large, closely spaced sidewall slits 148 can result in a highly flexible coupler tube having a small radius of curvature R2 such as illustrated in FIG. 1. As the sidewall slit spacing becomes greater and/or sidewall slit size becomes smaller, the flexibility of the coupler tube can decrease resulting in a radius of curvature R2 that is larger (e.g. FIGs. 2 and 3).

The invention contemplates variations in the plurality of sidewall slits 148 of the coupler tube 146, specifically varying pitch, size, geometry, and the like of the plurality of sidewall slits 148 to achieve desired deflection of the coupler tube 146. As one skilled in the pertinent art will appreciate, the adjustment of certain parameters of the sidewall slits 148 to achieve desired deflection of the coupler tube 146 can be advantageous in that it can allow the end effector 110 to reach difficult to access anatomies. For example, as mentioned previously, by varying the pitch or size of the sidewall slits 148 in the coupler tube 146, the coupler tube 146 can achieve a tighter radius of curvature R2. Due to the coupler tube 146 having a tighter radius of curvature R2, such as shown in FIG. 1, the end effector 110 can achieve greater efficacy when implemented in difficult to reach anatomies based at least in part on the deflective characteristics of the coupler tube 146. In another example, the plurality of sidewall slits 148 can be made into a pattern of circular slits that can liken the coupler tube 146 to a spring. By using the pattern of circular slits for the sidewall slits 148, the coupler tube 146 can use spring potential energy, when deflected, to reach difficult to access anatomies and can therein increase the efficacy of the end effector 110.

When the first puller wire 124 is retracted, the distal portion 114 of the elongated shaft 109 can bend to the right in a mirror image to FIGs. 1-3. The dimensions of the sidewall slits 148 can be selected to result in the coupler tube having a predetermined second radius of curvature R2. The sidewall slits 148 can further allow for the radius of curvature R2 to be different for the first puller wire 124 compared to the second puller wire 126. For instance, consider that the distal portion 114 of the elongated shaft 109 curves to the right to radius R1 for a given tension in the first puller wire 124; and the distal portion 114 curves to the same radius R1 to the left when the same tension is applied instead to the second puller wire 126. In this example, the second radius of curvature R2 of the coupler tube 146 need not be the same when the first puller wire 124 is under tension compared to when the second puller wire 126 is under tension. This can be accomplished by having a different slit pattern proximal of the first anchor 125 compared to a slit pattern proximal of the second anchor 128. This can also be accomplished by placing one of the anchors 125, 128 proximal in relation to the other anchor.

A control 111 (FIG. 7), illustrated as a knob, can be linked to at least one of the first or second puller wires 124, 126. The control 111 can configured to retract one or more puller wires such as the puller wires 124, 126 (FIGs. 5 and 6). Preferably, the controller is configured to retract the first puller wire 124 separately from the second puller wire 126 such that the first puller wire 124 and the second puller wire 126 are not retracted simultaneously with each other.

Referring again to FIG. 7, the end effector 110 can have a plurality of loop members 101, 102, 103 that overlap at a common distal vertex and are joined at the distal vertex with a mechanical linkage 150. The end effector 110 can include an electrode assembly that can be used for mapping and/or ablation medical procedures. The electrode assembly can be arranged in a planar grid shape, a sphere shape, a helical shape, a circular shape, a plurality of radial spines, a single irrigated tip electrode, a contact force sensor, and the like. The illustrated end effector 110 is planar and can further be configured similarly as disclosed in U.S. Patent Publication No. 2021/0369339, incorporated by reference herein.

The end effector 110 can be collapsed (compressed approximately perpendicular to and toward the longitudinal axis L-L) to fit within a guiding sheath or catheter (not illustrated). The elongated shaft 109 can be pushed distally to move the end effector 110 distally through the guiding sheath. The end effector 110 can be moved to exit a distal end of the guiding sheath via manipulation of the elongated shaft 109 and/or control handle 116. An example of a suitable guiding sheath for this purpose is the Preface Braided Guiding Sheath, commercially available from Biosense Webster, Inc. (Irvine, California, USA).

A distal electrode 138D and a proximal electrode 138P are positioned near the distal portion 115 of the elongated shaft 109. The electrodes 138D and 138P can be configured to cooperate (e.g. by masking of a portion of one electrode and masking a different portion on the other electrode) to define a referential electrode (an electrode that is not in contact with tissues). One or more impedance sensing electrodes 138R can be configured to allow for location sensing via impedance location sensing technique, as described in US Patent Nos. 5,944,022; and 5,983,126, which is incorporated herein by reference.

The configuration of the coupler tube 46 illustrated in FIGs. 5 and 6 can be used in high density diagnostic catheters having an end effector with multiple electrodes. The catheter 100 illustrated in FIG. 7 is one example. Other end effector designs with one or more spines carrying electrodes such as a lasso, ray, basket shape, etc. can be utilized in place of the end effector 110 illustrated in FIG. 7. Existing catheters with these shapes can be modified to include the coupler tube 46 illustrated herein to get additional shaft curvature compared to existing designs. Such catheters can be navigated using existing techniques, and then selectively bent additional degrees of curvature for additional reach as needed. This feature can be an improvement on existing catheters in which the point of deflection is too proximal in certain applications and it is difficult to reach certain anatomies with existing designs.

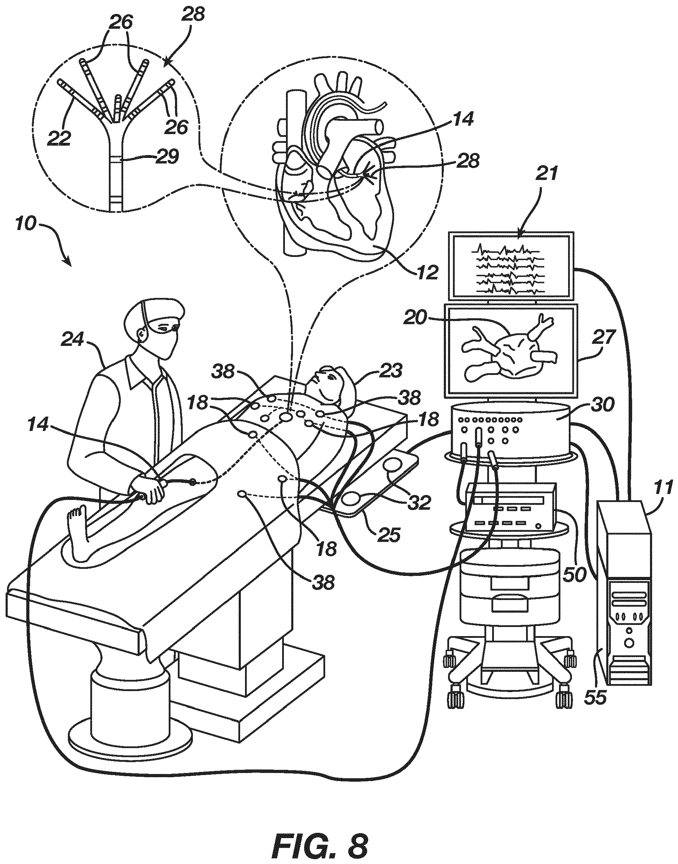

FIG. 8 is an illustration showing an example catheter-based electrophysiology mapping and ablation system 10. The system 10 includes multiple catheters, which are percutaneously inserted by a physician 24 through the patient's vascular system into a chamber or vascular structure of a heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters for both sensing and ablating. An example catheter 14 that is configured for sensing IEGM is illustrated herein. The physician 24 brings a distal tip 28 of the catheter 14 into contact with the heart wall for sensing a target site in the heart 12. For ablation, the physician 24 would similarly bring a distal end of an ablation catheter to a target site for ablating.

The shaft of the catheter 14 can be configured similarly to the shaft 109 of the example catheters 100 illustrated in FIGs. 1-4 and 7. The shaft of the catheter 14 can include a coupler tube 46 and puller wires 124, 126 configured similarly to one of the example catheters illustrated in FIGs. 1-4 and can include features of the coupler tube 46 illustrated in FIGs. 5 and 6. Likewise, any of the example catheters illutrated in FIGs. 1-4 and 7 can be used in place of catheter 14.

The illustrated catheter 14 is an exemplary catheter that includes one and preferably multiple electrodes 26 optionally distributed over a plurality of spines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

A magnetic based position sensor 29 may be operated together with a location pad 25 including a plurality of magnetic coils 32 configured to generate magnetic fields in a predefined working volume. Real time position of a distal tip 28 of the catheter 14 may be tracked based on magnetic fields generated with a location pad 25 and sensed by a magnetic based position sensor 29. Likewise, the position sensor 142 illustrated in FIG. 5 can be used in a similar manner as position sensor 29 illustrated in FIG. 8. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091 incorporated by reference herein.

The system 10 includes one or more electrode patches 38 positioned for skin contact on the patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182 incorporated by reference herein.

A recorder 11 displays electrograms 21 captured with body surface ECG electrodes 18 and intracardiac electrograms (IEGM) captured with electrodes 26 of the catheter 14. The recorder 11 may include pacing capability for pacing the heart rhythm and/or may be electrically connected to a standalone pacer.

Likewise, electrodes 107 of the catheter 100 illustrated in FIG. 7 can be used in a similar manner as the end effector electrodes 26 illustrated in FIG. 8.

The system 10 can include an ablation energy generator 50 that is adapted to conduct ablative energy to one or more of electrodes at a distal tip of a catheter configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

A patient interface unit (PIU) 30 is an interface configured to establish electrical communication between catheters, electrophysiological equipment, power supply and a workstation 55 for controlling operation of system 10. Electrophysiological equipment of the system 10 may include for example, multiple catheters, a location pad 25, body surface ECG electrodes 18, electrode patches 38, an ablation energy generator 50, and a recorder 11. Optionally and preferably, the PIU 30 includes processing capability for implementing real-time computations of location of the catheters and for performing ECG calculations.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

Thus, in use, the physician 24 can introduce the distal end of the catheter 100 (illustrated in FIG. 7 with variations thereof in FIGs. 1-4) into the heart 12 of a patient and manipulate its position via the handle 116. The control 111, illustrated in FIG. 7 as a knob, can be linked to at least one of the first or second puller wires 124, 126 (FIGs. 5 and 6). As the physician 24 positions the end effector 110 to contact the heart tissue, she can manipulate the control 111 to deflect the end effector 110 from the longitudinal axis L-L to get a proper positioning of the end effector 110 on the tissue surface.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

The following clauses list non-limiting embodiments of the disclosure:
1. A catheter comprising:
   an elongated shaft extending along a longitudinal axis;
   an electrode assembly disposed distal of the elongated shaft;
   a coupler tube disposed between the elongated shaft and the electrode assembly; and
   a puller wire extending through the elongated shaft, comprising a distal end joined to a sidewall of the coupler tube so that translation of the puller wire along the longitudinal axis deflects a distal portion of the elongated shaft away from the longitudinal axis so that the distal portion defines a first radius of curvature and the coupler tube defines a second radius of curvature.
2. The catheter of clause 1, wherein the distal portion of the elongated shaft, deflectable from the longitudinal axis by the puller wire, includes a length of the elongated shaft up to a proximal end of the coupler tube.
3. The catheter of clause 1 or 2, the puller wire being configured to deflect at least a portion of the coupler tube.
4. The catheter of clause 3, the first radius of curvature being less than the second radius of curvature.
5. The catheter of clause 3, the first radius of curvature being approximately equal to the second radius of curvature.
6. The catheter of clause 3, the first radius of curvature being greater than the second radius of curvature.
7. The catheter of any one of clauses 3-6, the coupler tube comprising a plurality of sidewall slits configured to facilitate deflection of the coupler tube.
8. The catheter of clause 7, the sidewall slits comprising a plurality of slits each orthogonal to the longitudinal axis.
9. The catheter of clause 7 or 8, the sidewall slits comprising a plurality of slits aligned along the longitudinal axis with the distal end of the puller wire.
10. The catheter of any one of clauses 3-9, the coupler tube comprising a material with a durometer configured to allow the coupler tube to deflect.
11. The catheter of any one of clauses 1-10, the distal end of the puller wire being affixed through the sidewall of the coupler tube.
12. The catheter of any one of clauses 1-11, the coupler tube comprising a single lumen.
13. The catheter of any one of clauses 1-12, the elongated shaft comprising a distal extension disposed within a proximal portion of the coupler tube.
14. The catheter of any one of clauses 1-13, the electrode assembly comprising a support frame comprising a proximal portion disposed within a distal portion of the coupler tube.
15. The catheter of clause 14, further comprising:
   a disk affixed within the distal portion of the coupler tube, joined to the proximal portion of the support frame, and comprising one or more lumens with electrical conductors extending therethrough.
16. The catheter of any one of clauses 1-15, further comprising:
   elongated conductors extending from the electrode assembly, the coupler tube, and the elongated shaft.
17. The catheter of any one of clauses 1-16, further comprising:
   an electromagnetic position sensor disposed within the coupler tube.
18. The catheter of any one of clauses 1-17, further comprising:
   a pair of puller wires each extending through the elongated shaft, each comprising a distal end affixed to the coupler tube, and each being configured to deflect a distal portion of the elongated shaft from the longitudinal axis.
19. The catheter of clause 18, the distal ends of the puller wires being affixed on opposite sides of the coupler tube.
20. The catheter of any one of clauses 1-19, the electrode assembly comprising an array of electrodes arranged in a planar grid.
21. The catheter of any one of clauses 1-19, the electrode assembly comprising an array of electrodes arranged in a sphere.
22. The catheter of any one of clauses 1-19, the electrode assembly comprising a single irrigated tip electrode and a contact force sensor.
23. The catheter of any one of clauses 1-19, the electrode assembly comprising an array of electrodes arranged in a helical or circular shape.
24. The catheter of any one of clauses 1-19, the electrode assembly comprising a plurality of radial spines.
25. The catheter of any one of clauses 1-24, further comprising:
   a handle proximal of the elongated shaft and configured to provide tension to the puller wire.
26. The catheter of any one of clauses 1-25, a proximal portion of the elongated shaft comprising a stiffening tube configured to inhibit deflection of the proximal portion of the elongated shaft by the puller wire.
27. A method of manufacturing a catheter, the method comprising:
   joining a puller wire to a sidewall of a coupler tube;
   affixing an electrode assembly to a distal end of the coupler tube; and
   affixing an elongated shaft to a proximal end of the coupler tube such that the puller wire extends through the elongated shaft and so that translation of the puller wire along a longitudinal axis deflects a distal portion of the elongated shaft away from the longitudinal axis so that the distal portion defines a first radius of curvature and the coupler tube defines a second radius of curvature.
28. The method of clause 27, wherein the puller wire is configured to deflect a distal portion of the elongated shaft including a length of the elongated shaft up to a proximal end of the coupler tube.
29. The method of clause 27 or 28, wherein the puller wire is configured to deflect at least a portion of the coupler tube.
30. The method of any one of clauses 27-29, further comprising:
   selecting a material durometer of the coupler tube to allow the coupler tube to deflect.
31. The method of any one of clauses 27-30, further comprising:
   cutting sidewall slits in the coupler tube configured to facilitate deflection of the coupler tube.
32. The method of any one of clauses 27-31, wherein joining the puller wire to the sidewall of the coupler tube comprises welding the puller wire through the sidewall of the coupler tube.

## Claims

1. A catheter comprising:
an elongated shaft extending along a longitudinal axis;
an electrode assembly disposed distal of the elongated shaft;
a coupler tube disposed between the elongated shaft and the electrode assembly; and
a puller wire extending through the elongated shaft, comprising a distal end joined to a sidewall of the coupler tube so that translation of the puller wire along the longitudinal axis deflects a distal portion of the elongated shaft away from the longitudinal axis so that the distal portion defines a first radius of curvature and the coupler tube defines a second radius of curvature.

2. The catheter of claim 1, wherein the distal portion of the elongated shaft, deflectable from the longitudinal axis by the puller wire, includes a length of the elongated shaft up to a proximal end of the coupler tube.

3. The catheter of claim 1 or claim 2, the puller wire being configured to deflect at least a portion of the coupler tube.

4. The catheter of claim 3, the first radius of curvature being i) less than the second radius of curvature, ii) approximately equal to the second radius of curvature, or iii) greater than the second radius of curvature.

5. The catheter of claim 3 or claim 4, the coupler tube comprising a plurality of sidewall slits configured to facilitate deflection of the coupler tube.

6. The catheter of claim 5, the sidewall slits comprising a plurality of slits each orthogonal to the longitudinal axis, and/or the sidewall slits comprising a plurality of slits aligned along the longitudinal axis with the distal end of the puller wire.

7. The catheter of any of claims 3 to 6, the coupler tube comprising a material with a durometer configured to allow the coupler tube to deflect.

8. The catheter of any preceding claim, the distal end of the puller wire being affixed through the sidewall of the coupler tube.

9. The catheter of any preceding claim, the coupler tube comprising a single lumen.

10. The catheter of any preceding claim, the elongated shaft comprising a distal extension disposed within a proximal portion of the coupler tube.

11. The catheter of any preceding claim, the electrode assembly comprising a support frame comprising a proximal portion disposed within a distal portion of the coupler tube.

12. The catheter of claim 11, further comprising:
a disk affixed within the distal portion of the coupler tube, joined to the proximal portion of the support frame, and comprising one or more lumens with electrical conductors extending therethrough.

13. The catheter of any preceding claim, further comprising:
a pair of puller wires each extending through the elongated shaft, each comprising a distal end affixed to the coupler tube, and each being configured to deflect a distal portion of the elongated shaft from the longitudinal axis.

14. The catheter of claim 13, the distal ends of the puller wires being affixed on opposite sides of the coupler tube.

15. The catheter of any preceding claim, the electrode assembly comprising i) an array of electrodes arranged in a planar grid, ii) an array of electrodes arranged in a sphere, or iii) a single irrigated tip electrode and a contact force sensor.
